# EUROPEAN PATENT APPLICATION

(11) **EP 4 509 489 A1**
(43) Date of publication of application: **19.02.2025**
(21) Application number: 22937531.6
(22) Date of filing: 28.12.2022
(51) Int. Cl.: C07C 1/04, C07C 9/08, C07C 9/10, B01J 37/16, C10L 3/12, B01J 29/46

(54) **CATALYST FOR LIQUEFIED PETROLEUM GAS SYNTHESIS, AND METHOD FOR PRODUCING LIQUEFIED PETROLEUM GAS**

(30) Priority: 15.04.2022 JP 2022067954
(71) Applicant: FURUKAWA ELECTRIC CO., LTD., Tokyo 100-8322 (JP)
(72) Inventor: FUJIKAWA, Takashi, Tokyo 100-8322 (JP); IWANO, Yuki, Tokyo 100-8322 (JP); TAKAHASHI, Hiroko, Tokyo 100-8322 (JP); BAMBA, Yuichiro, Tokyo 100-8322 (JP); KAWAMATA, Yuki, Tokyo 100-8322 (JP); LEE, Yu, Tokyo 100-8322 (JP); HIRANO, Junya, Tokyo 100-8322 (JP); FUKUSHIMA, Masayuki, Tokyo 100-8322 (JP)
(74) Representative: Winter, Brandl - Partnerschaft mbB
(86) International application number: PCT/JP2022/048619
(87) International publication number: WO 2023/199557

(57) **Abstract**

This catalyst for liquefied petroleum gas synthesis includes a Cu-Zn based catalytic material and an MFI type zeolite catalytic material supporting Pt, the Cu-Zn based catalytic material containing copper oxide, zinc oxide, aluminium oxide, and zirconium oxide, a mass (M(ZrO₂)) of zirconium oxide in the Cu-Zn based catalytic material being more than 0 mass% and 6.5 mass% or less based on a mass (M1) of the Cu-Zn based catalytic material, and the MFI type zeolite catalyst material containing more than 0 mass% and less than 4.5 mass% of P.

## Description

### TECHNICAL FIELD

The present disclosure relates to a catalyst for liquefied petroleum gas synthesis and a method for producing liquefied petroleum gas.

### BACKGROUND ART

Liquefied petroleum gas (LPG) containing propane and butane as main components exists in oil fields and natural gas fields, in a state of being mixed with impurity gases such as methane and ethane. After transferring such gases to aboveground facilities, propane and butane are separated and recovered from the gases, and further, impurities such as sulfur and mercury are removed to obtain the liquefied petroleum gas.

Furthermore, the liquefied petroleum gas is also contained in crude oil. Therefore, it is also possible to obtain the liquefied petroleum gas by separating and extracting propane and butane during a refining process at oil refineries.

Moreover, methods for producing the liquefied petroleum gas from synthesis gas of carbon monoxide and hydrogen have been studied. For instance, Patent Documents 1 to 4 disclose production of liquefied petroleum gas using a methanol synthesizing catalyst and a palladium-supporting zeolite catalyst.

In the production of liquefied petroleum gas, a high CO conversion rate is desirable for increasing the productivity of liquefied petroleum gas. Also, it is desirable that a total yield of propane and butane is high in the production of liquefied petroleum gas. It is particularly desirable that a yield of propane is high among liquefied petroleum gas because propane is easily vaporized and therefore can be easily used as a fuel even in e.g. cold regions.

### Citation List

### Patent Document

Patent Document 1: Japanese Patent No. 5405103
Patent Document 2: Japanese Patent No. 5086658
Patent Document 3: Japanese Patent No. 4989650
Patent Document 4: Japanese Unexamined Patent Application, Publication No. 2019-37939

### DISCLOSURE OF THE INVENTION

### Problems to be Solved by the Invention

An object of the present disclosure is to provide a catalyst for liquefied petroleum gas synthesis and a method for producing liquefied petroleum gas capable of improving a CO conversion rate, a total yield of propane and butane, and a yield of propane.

### Means for Solving the Problems

[1] A catalyst for liquefied petroleum gas synthesis, including a Cu-Zn based catalytic material and an MFI type zeolite catalytic material supporting Pt, in which the Cu-Zn based catalytic material contains copper oxide, zinc oxide, aluminium oxide, and zirconium oxide, a mass (M(ZrO₂)) of zirconium oxide in the Cu-Zn based catalytic material is more than 0 mass% and 6.5 mass% or less based on a mass (M1) of the Cu-Zn based catalytic material, and the MFI type zeolite catalytic material contains more than 0 mass% and less than 4.5 mass% of P.
[2] The catalyst for liquefied petroleum gas synthesis as described in [1], in which a mass (M(Al₂O₃)) of the aluminium oxide in the Cu-Zn based catalytic material is 3.5 mass% or more and 8.0 mass% or less based on the mass (M1) of the Cu-Zn based catalytic material.
[3] The catalyst for liquefied petroleum gas synthesis as described in [1] or [2], in which a ratio (M1/(M1+M2)) of the mass (M1) of the Cu-Zn based catalytic material to a total mass (M1+M2) of the mass (M1) of the Cu-Zn based catalytic material and the mass (M2) of the MFI type zeolite catalytic material is 0.30 or higher and 0.95 or lower.
[4] The catalyst for liquefied petroleum gas synthesis as described in any one of [1] to [3], in which a mass (M(Pt)) of Pt in the MFI type zeolite catalytic material is 0.1 mass% or more and 10.0 mass% or less based on the mass (M2) of the MFI type zeolite catalytic material.
[5] The catalyst for liquefied petroleum gas synthesis as described in any one of [1] to [4], in which the Cu-Zn based catalytic material and the MFI type zeolite catalytic material exist independently from each other, and both of the Cu-Zn based catalytic material and the MFI type zeolite catalytic material are in a form of granulated powder or molded body.
[6] A method for producing liquefied petroleum gas including: a reduction treatment step of reducing the catalyst for liquefied petroleum gas synthesis as described in any one of [1] to [5]; a supply step of supplying carbon monoxide and hydrogen to the catalyst for liquefied petroleum gas synthesis reduced in the reduction treatment step; and a synthesis step of synthesizing liquefied petroleum gas by reacting carbon monoxide and hydrogen supplied in the supply step using the catalyst for liquefied petroleum gas synthesis subjected to the reduction treatment.
[7] The method for producing liquefied petroleum gas as described in [6], in which a gas hourly space velocity (GHSV) for supplying carbon monoxide and hydrogen is 500/h or higher and 20000/h or lower in the supply step.
[8] The method for producing liquefied petroleum gas as described in [6] or [7], in which carbon monoxide and hydrogen are reacted at a temperature of 260°C or higher and 330°C or lower in the synthesis step.
[9] The method for producing liquefied petroleum gas as described in any one of [6] to [8], in which carbon monoxide and hydrogen are reacted under a pressure of 2.0 MPa or higher and 6.0 MPa or lower in the synthesis step.

### Effects of the Invention

According to the present disclosure, it is possible to provide a catalyst for liquefied petroleum gas synthesis and a method for producing liquefied petroleum gas, which can improve a CO conversion rate, a total yield of propane and butane, and a yield of propane.

### BRIEF DESCRIPTION OF THE DRAWINGS

Fig. 1 is a diagram illustrating results of CO conversion rate in Examples and Comparative Examples.
Fig. 2 is a diagram illustrating results of total yield of propane and butane in Examples and Comparative Examples.
Fig. 3 is a diagram illustrating results of yield of propane in Examples and Comparative Examples.
Fig. 4 is a diagram illustrating results of yield of butane in Examples and Comparative Examples.
Fig. 5 is a diagram illustrating results of volume ratio of the propane yield to the total yield of propane and butane in Examples and Comparative Examples.

### PREFERRED MODE FOR CARRYING OUT THE INVENTION

A detailed description based on the embodiments follows.

The present inventors, after intensive research, found that a CO conversion rate, a total yield of propane and butane, and a propane yield could be improved by a catalyst including a Cu-Zn based catalytic material and an MFI type zeolite catalytic material supporting Pt, in which the Cu-Zn based catalytic material contained copper oxide, zinc oxide, aluminium oxide, and zirconium oxide, a mass (M(ZrO₂)) of zirconium oxide in the Cu-Zn based catalytic material was more than 0 mass% and 6.5 mass% or less based on a mass (M1) of the Cu-Zn based catalytic material, and the MFI type zeolite catalytic material contained more than 0 mass% and less than 4.5 mass% of P. Such findings led to the completion of the present disclosure.

The catalyst for liquefied petroleum gas synthesis of the present embodiment includes a Cu-Zn based catalytic material and an MFI type zeolite catalytic material supporting Pt (hereinafter, also simply referred to as MFI type zeolite catalytic material). The Cu-Zn based catalytic material contains copper oxide, zinc oxide, aluminium oxide, and zirconium oxide. A mass (M(ZrO₂)) of zirconium oxide in the Cu-Zn based catalytic material is more than 0 mass% and 6.5 mass% or less based on a mass (M1) of the Cu-Zn based catalytic material. The MFI type zeolite catalytic material contains more than 0 mass% and less than 4.5 mass% of P.

As mentioned above, the catalyst for liquefied petroleum gas synthesis of the present embodiment includes a Cu-Zn based catalytic material and an MFI type zeolite catalytic material. The catalyst for liquefied petroleum gas synthesis can synthesize liquefied petroleum gas from carbon monoxide and hydrogen. The liquefied petroleum gas synthesized by the catalyst for liquefied petroleum gas synthesis of the present embodiment contains propane and butane, with propane being more predominant than butane. In the liquefied petroleum gas synthesized by the catalyst for liquefied petroleum gas synthesis of the present embodiment, a ratio of the combined total of propane and butane to the liquefied petroleum gas is, for example, 28 Cmol% or higher. A ratio of propane synthesized by the catalyst for liquefied petroleum gas synthesis of the present embodiment is 20 Cmol% or higher. Moreover, in the liquefied petroleum gas synthesized by the catalyst for liquefied petroleum gas synthesis of the present embodiment, a ratio of propane to the combined total of propane and butane is, for example, 80 mol% or higher by volume.

The Cu-Zn based catalytic material composing the catalyst for liquefied petroleum gas synthesis has a function as a liquefied petroleum gas precursor synthesizing catalyst to synthesize liquefied petroleum gas precursors such as methanol and dimethyl ether from carbon monoxide and hydrogen.

In the present embodiment, regarding a ratio (M1/(M1+M2)) of the mass (M1) of the Cu-Zn based catalytic material to the total mass (M1+M2) of the mass (M1) of the Cu-Zn based catalytic material and the mass (M2) of the MFI type zeolite catalytic material (hereinafter, also simply referred to as a mass ratio of the Cu-Zn based catalytic material), the lower limit value is preferably 0.30 or higher, more preferably 0.35 or higher, even more preferably 0.40 or higher, and the upper limit value is preferably 0.95 or lower, more preferably 0.80 or lower, even more preferably 0.70 or lower, particularly preferably 0.65 or lower, most preferably 0.60 or lower. When the mass ratio (M1/(M1+M2)) of the above Cu-Zn based catalytic material is 0.30 or higher and 0.95 or lower, liquefied petroleum gas can be efficiently synthesized from carbon monoxide and hydrogen.

The Cu-Zn based catalytic material composing the catalyst for liquefied petroleum gas synthesis contains copper oxide, zinc oxide, aluminium oxide, and zirconium oxide. The mass (M(ZrO₂)) of zirconium oxide in the Cu-Zn based catalytic material is more than 0 mass% and 6.5 mass% or less based on the mass (M1) of the Cu-Zn based catalytic material.

Among catalysts for synthesizing liquefied petroleum gas precursors, a catalyst containing copper oxide, zinc oxide, and aluminium oxide has excellent performance to synthesize liquefied petroleum gas precursors. In such a catalyst containing copper oxide, zinc oxide, and aluminium oxide, a part of aluminium oxide is replaced with a specific amount of zirconium oxide, i.e. replaced with zirconium oxide in such an amount that the mass (M(ZrO₂)) of zirconium oxide in the Cu-Zn based catalytic material is more than 0 mass% and 6.5 mass% or less based on the mass (M1) of the Cu-Zn based catalytic material, and furthermore the Cu-Zn based catalytic material is used together with the MFI type zeolite catalytic material containing a predetermined amount of P and supporting Pt as described later in detail, to improve the CO conversion rate, the total yield of propane and butane, and the propane yield.

At the present time, the present inventors assume, as follows, the reason why the CO conversion rate, the total yield of propane and butane, and the propane yield can be improved by using a catalyst containing copper oxide, zinc oxide, aluminium oxide, and the above-specified amount of zirconium oxide as the Cu-Zn based catalytic material composing the catalyst for liquefied petroleum gas synthesis. When the catalyst contains aluminium oxide, the dispersibility of copper oxide and zinc oxide can be improved and an interface between copper and zinc oxide, which is speculated to be an active point, is increased, and when the catalyst contains a specific amount of zirconium oxide, oxidation of copper is suppressed. Thus, the CO conversion rate, especially the conversion rate into propane, is improved, and therefore it is presumed that the propane and butane total yield and the propane yield can be improved. On the other hand, when the catalyst does not contain zirconium oxide, when the catalyst contains zirconium oxide but in an amount outside the above-specified range, or when the catalyst does not contain aluminium oxide, the CO conversion rate, the propane and butane total yield, and the propane are decreased.

The mass (M(ZrO₂)) of zirconium oxide in the Cu-Zn based catalytic material is more than 0 mass% and 6.5 mass% or less based on the mass (M1) of the Cu-Zn based catalytic material, and the lower limit value is preferably 2.0 mass% or more, more preferably 3.5 mass% or more, even more preferably 4.0 mass% or more, most preferably 4.5 mass% or more, and the upper limit value is preferably 6.0 mass% or less, more preferably 5.0 mass% or less.

In the Cu-Zn based catalytic material, regarding the total mass (M(ZrO₂)+M(Al₂O₃)) of the mass (M(ZrO₂)) of zirconium oxide and the mass (M(Al₂O₃)) of aluminium oxide based on the mass (M1) of the Cu-Zn based catalytic material, the lower limit value is preferably 5.0 mass% or more, more preferably 7.5 mass% or more, and the upper limit value is preferably 15.0 mass% or less, more preferably 12.5 mass% or less.

In the Cu-Zn based catalytic material, regarding the mass (M(Al₂O₃)) of aluminium oxide based on the mass (M1) of the Cu-Zn based catalytic material, the lower limit value is preferably 3.5 mass% or more, more preferably 4.0 mass% or more, even more preferably 4.5 mass% or more, and the upper limit value is preferably 8.0 mass% or less, more preferably 7.0 mass% or less, even more preferably 6.0 mass% or less.

In the Cu-Zn based catalytic material, regarding the mass (M(CuO)) of copper oxide (CuO) based on the mass (M1) of the Cu-Zn based catalytic material, the lower limit value is preferably 50 mass% or more, more preferably 55 mass% or more, even more preferably 60 mass% or more, and the upper limit value is preferably 75 mass% or less, more preferably 70% or less, even more preferably 65 mass% or less.

In the Cu-Zn based catalytic material, regarding the mass (M(ZnO)) of zinc oxide (ZnO) based on the mass (M1) of the Cu-Zn based catalytic material, the lower limit value is preferably 15 mass% or more, more preferably 20 mass% or more, even more preferably 25 mass% or more, and the upper limit value is preferably 40 mass% or less, more preferably 35% or less, even more preferably 30 mass% or less.

In the Cu-Zn based catalytic material, the ratio (M(ZnO)/M(CuO)) of the mass (M(ZnO)) of zinc oxide (ZnO) to the mass (M(CuO)) of copper oxide (CuO) is preferably 0.40 or higher and 0.60 or lower, more preferably 0.45 or higher and 0.55 or lower.

The Cu-Zn based catalytic material may contain gallium oxide, indium oxide, and the like, in addition to copper oxide, zinc oxide, aluminium oxide, and zirconium oxide. When a catalyst containing gallium oxide, indium oxide, and the like, in addition to copper oxide, zinc oxide, gallium oxide, and zirconium oxide is used as the Cu-Zn based catalytic material composing the catalyst for liquefied petroleum gas synthesis, the CO conversion rate, the propane and butane total yield, and the propane yield can be improved as in the case of using the catalyst of the present embodiment containing copper oxide, zinc oxide, aluminium oxide, and zirconium oxide.

The presence or absence and the content ratios of copper oxide, zinc oxide, aluminium oxide, and zirconium oxide in the Cu-Zn based catalytic material can be measured by ICP-OES (Inductively Coupled Plasma Optical Emission Spectroscopy).

The MFI type zeolite catalytic material composing the catalyst for liquefied petroleum gas synthesis synthesizes liquefied petroleum gas from the liquefied petroleum gas precursors generated by the Cu-Zn based catalytic material. In the present embodiment, the synthesized liquefied petroleum gas contains propane and butane, with propane being more predominant than butane.

Regarding the zeolite catalytic material that composes the catalyst for liquefied petroleum gas synthesis, the type of zeolite is MFI type. The MFI type zeolite catalytic material has a smaller pore diameter compared to beta type zeolite, and therefore it is assumed that propane can be synthesized more efficiently than butane or the like among components of liquefied petroleum gas and the propane yield can be enhanced.

Also, the MFI type zeolite catalytic material supports Pt (platinum). Since the MFI type zeolite catalytic material supporting Pt can efficiently react the liquefied petroleum gas precursors, potentially leading to a higher propane yield.

In the MFI type zeolite catalytic material, regarding the mass (M(Pt)) of Pt based on the mass (M2) of the MFI type zeolite catalytic material, the lower limit value is preferably 0.1 mass% or more, more preferably 0.2 mass% or more, even more preferably 0.3 mass% or more, and the upper limit value is preferably 10.0 mass% or less, more preferably 5.0 mass% or less, even more preferably 3.0 mass% or less.

When the above mass (M(Pt)) of Pt based on the mass (M2) of the MFI type zeolite catalytic material is 0.1 mass% or higher, an active point attributed to Pt is generated, resulting in efficient synthesis of liquefied petroleum gas. Moreover, when the above mass (M(Pt)) of Pt based on the mass (M2) of the MFI type zeolite catalytic material is 10.0 mass% or less, decrease in the dispersibility of active metals resulting from Pt aggregation or the like can be suppressed, and efficient synthesis of liquefied petroleum gas can be maintained to an extent not exceeding a limit value of the active metal content allowing efficient dispersion, while suppressing the increase in material costs due to excessive Pt.

The MFI type zeolite catalytic material may support not only Pt but also a platinum group element such as pd (palladium), rhodium (Rh), or ruthenium (Ru). The noble metal may be one type or two or more types. When there are two or more types of noble metals, the noble metals may be supported by the MFI type zeolite catalytic material in any state, and, for example, each noble metal may be mixed in an elemental metal state, or the noble metals may be alloyed, or the noble metals may be mixed in a combined state of elemental metals and alloys.

ICP-OES (Inductively Coupled Plasma Optical Emission Spectroscopy) can measure whether or not the noble metal such as Pd or Pt are supported by the MFI type zeolite catalytic material, and the mass (M(Pt)) of Pt based on the mass (M2) of the above MFI type zeolite catalytic material.

Also, the MFI type zeolite catalytic material contains more than 0 mass% and less than 4.5 mass% of P (phosphorus). When the MFI type zeolite catalytic material contains P in an amount within the above range, acid sites (solid acid sites) of the MFI type zeolite catalytic material increase and simultaneously change into weak acid sites, thereby especially the propane yield can be improved, and, for example, a ratio of propane to the combined total of propane and butane can be increased. When the MFI type zeolite catalytic material contains P, it is assumed that P binds to O (oxygen) bound to Si and O bound to Al on the surface of the zeolite catalytic material, as shown in Formula (1) below.

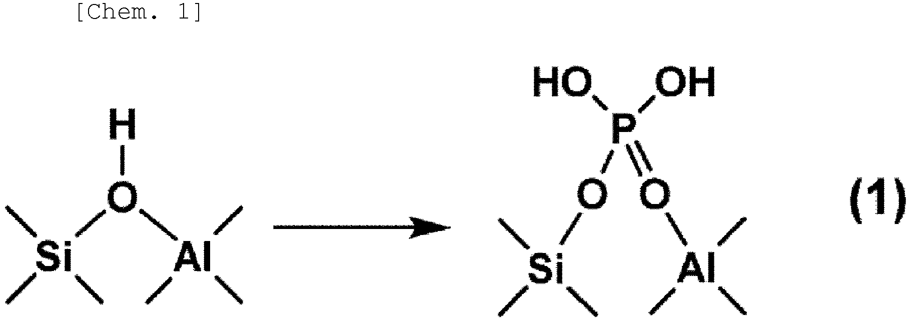

In the MFI type zeolite catalytic material, regarding the ratio (mass%) ((M(P)/M2)×100)) (hereinafter, also referred to as "P content ratio") of the mass (M(P)) of P to the mass (M2) of the MFI type zeolite catalytic material, the lower limit value is more than 0 mass%, preferably 0.5 mass% or more, more preferably 1.0 mass% or more, even more preferably 1.5 mass% or more, and the upper limit value is less than 4.5 mass%, preferably 4.0 mass% or less, more preferably 3.0 mass% or less, even more preferably 2.5 mass% or less. When the above mass (M(P)) of P is more than 0 mass% based on the mass (M2) of the MFI type zeolite catalytic material, the CO conversion rate, the propane and butane total yield, and the propane yield can be improved. When the above mass (M(P)) of P is less than 4.5 mass% based on the mass (M2) of the MFI type zeolite catalytic material, a decrease in the ratio of propane to the combined total of propane and butane, and a decrease in the propane or butane yield due to excessive P content can be suppressed.

The presence or absence of P, and the above content ratio of P in the MFI type zeolite catalytic material can be measured by ICP-OES (Inductively Coupled Plasma Optical Emission Spectroscopy).

Additionally, in the MFI type zeolite catalytic material, the ratio (molar number of SiO₂/molar number of Al₂O₃) (hereinafter, also simply referred to as molar ratio (SiO₂/Al₂O₃)) of the molar number of SiO₂ to the molar number of Al₂O₃ is preferably 20 or higher and 60 or lower. The MFI type zeolite catalytic material is an aluminosilicate. By substituting some of silicon atoms in the silicate forming the zeolite framework of the MFI type zeolite catalytic material with aluminium atoms, the aluminium atom becomes the acid site, and therefore the MFI type zeolite catalytic material exhibits the function as a solid acid.

When the above molar ratio (SiO₂/Al₂O₃) is 60 or lower, the acid sites of the MFI type zeolite catalytic material increase, this leads to an increase in the production amount of liquefied petroleum gas and also to an increase in the amount of propane contained in the liquefied petroleum gas due to efficient synthesis of propane. Also, when the above molar ratio (SiO₂/Al₂O₃) is 20 or higher, the MFI type zeolite catalytic material supporting Pt can be produced easily while maintaining a high production capability of liquefied petroleum gas and a high synthesis capability of propane.

From the perspective of easily producing the above MFI type zeolite catalytic material, the above molar ratio (SiO₂/Al₂O₃) is preferably 20 or higher, more preferably 25 or higher, even more preferably 30 or higher. From the perspective of above high catalytic performance, the above molar ratio (SiO₂/Al₂O₃) is preferably 60 or lower, more preferably 50 or lower, even more preferably 40 or lower.

The above molar ratio (SiO₂/Al₂O₃) can be measured by ICP-OES (Inductively Coupled Plasma Optical Emission Spectroscopy).

Furthermore, the solid acid amount of the MFI type zeolite catalytic material is e.g. 0.6 mmol/g or more, preferably 0.8 mmol/g or more. When the solid acid amount is 0.6 mmol/g or more, the MFI type zeolite catalytic material supporting the noble metal can be produced easily while maintaining a high production capability of liquefied petroleum gas and a high synthesis capability of propane.

The above-mentioned solid acid amount can be measured by NH₃-TPD (Ammonia Temperature Programmed Desorption).

The Cu-Zn based catalytic material and the MFI type zeolite catalytic material preferably exist independently from each other, and both of the Cu-Zn based catalytic material and the MFI type zeolite catalytic material are preferably in the form of granulated powder or molded body. For the catalyst for liquefied petroleum gas synthesis, the Cu-Zn based catalytic material and the MFI type zeolite catalytic material are preferably not integrated (indistinctly integrated). The state of the Cu-Zn based catalytic material and the MFI type zeolite catalytic material may be in the form of granulated powder (powder. for example, particle size of 10⁻⁹ to 10⁻⁴ m) or granules having a particle size larger than the granulated powder, however, when the Cu-Zn based catalytic material is in a form of molded body containing the Cu-Zn based catalytic material and the MFI type zeolite catalytic material is in a form of molded body containing the MFI type zeolite catalytic material, the yield of propane and butane can be further increased. In other words, the catalyst for liquefied petroleum gas synthesis is preferably a mixture of the molded bodies containing the Cu-Zn based catalytic material and the molded bodies containing the MFI type zeolite catalytic material.

For the molded body containing the Cu-Zn based catalytic material, the content ratio of the Cu-Zn based catalytic material contained in the molded body is preferably 80 mass% or more, more preferably 90 mass% or more, even more preferably 95 mass% or more. When the above content ratio is 80 mass% or more, the liquefied petroleum gas precursors can be efficiently synthesized from carbon monoxide and hydrogen. The content ratio of the Cu-Zn based catalytic material contained in the molded body may be 100 mass%. Additionally, the above content ratio is preferably 98 mass% or less, more preferably 96 mass% or less, even more preferably 94 mass% or less. When the above content ratio is 98 mass% or less, the moldability and the mechanical strength of the molded body can be improved while maintaining efficient synthesis of the liquefied petroleum gas precursors.

The molded body containing the Cu-Zn based catalytic material may also contain various additives that improve the moldability or the mechanical strength, in addition to the Cu-Zn based catalytic material. Examples of such additives include molding binders such as graphite and carbon black.

For the molded body containing the MFI type zeolite catalytic material, the content ratio of the MFI type zeolite catalytic material contained in the molded body is preferably 70 mass% or more, more preferably 80 mass% or more, even more preferably 90 mass% or more. When the above content ratio is 70 mass% or more, liquefied petroleum gas can be efficiently synthesized from the liquefied petroleum gas precursors. The content ratio of the MFI type zeolite catalytic material contained in the molded body may be 100 mass%. The above content ratio is preferably 98 mass% or less, more preferably 96 mass% or less, even more preferably 94 mass% or less. When the above content ratio is 98 mass% or less, the moldability and the mechanical strength of the molded body can be improved while maintaining efficient synthesis of liquefied petroleum gas.

Similar to the molded body containing the Cu-Zn based catalytic material, the molded body containing the MFI type zeolite catalytic material may also include various additives that improves the moldability or the mechanical strength, in addition to the MFI type zeolite catalytic material. Examples of such additives include molding binders such as various clay binders, alumina-based binder, and silica-based binder. Examples of the various clay binders include kaolin-based binders, bentonite-based binders, talc-based binders, pyrophyllite-based binders, molysite-based binders, vermiculite-based binders, montmorillonite-based binders, chlorite-based binders, and halloysite-based binders. To effectively improve the catalyst activity and suppress the formation of catalyst poisoning substance such as coke, the molding binder is preferably a silica-based binder.

The shapes of the Cu-Zn based catalytic material and the MFI type zeolite catalytic material are not limited in particular. When the Cu-Zn based catalytic material or the MFI type zeolite catalytic material is in the form of molded body, desired shapes can be selected, such as cylindrical, clover-like, ring-like, spherical, and multi-holed shapes. In the case of cylindrical or clover-like shaped molded body, the molded body is preferably an extrusion-molded body.

Regarding the particle sizes of the molded body containing the Cu-Zn based catalytic material and the molded body containing the MFI type zeolite catalytic material, the lower limit value is preferably 200 µm or larger, more preferably 300 µm or larger, and the upper limit value is preferably 10 mm or smaller, more preferably 5 mm or smaller, even more preferably 3 mm or smaller. When the above particle size is 200 µm or larger, pressure loss within the reactor can be prevented. When the above particle size is 10 mm or smaller, the contact efficiency between the reactant and the catalyst for liquefied petroleum gas synthesis (Cu-Zn based catalytic material and MFI type zeolite catalytic material) in the reactor can be enhanced. The particle size can be measured by the dry sieving test method.

Furthermore, regarding the bulk densities of the molded body containing the Cu-Zn based catalytic material and the molded body containing the MFI type zeolite catalytic material, the lower limit value is preferably 0.5 g/cm³ or higher, and the upper limit value is preferably 1.5 g/cm³ or lower, more preferably 1.0 g/cm³ or lower. The bulk density can be measured using the sock loading bulk density measurement method with a measuring cylinder.

By using the catalyst for liquefied petroleum gas synthesis of the present embodiment, the CO conversion rate can be increased even at a low liquefied petroleum gas synthesis temperature (e.g. 330°C or lower), so that propane and butane, especially propane can be produced with a high yield.

In the synthesis of liquefied petroleum gas, the yield of liquefied petroleum gas such as propane can be increased by raising the synthesis temperature. In this case, when the above ratio (M1/(M1+M2)) is 0.70 or higher and 0.95 or lower, the catalyst for liquefied petroleum gas synthesis of the present embodiment can produce propane with a high yield even at a low liquefied petroleum gas synthesis temperature (e.g. 330°C or lower). The synthesis temperature refers to a temperature of the catalyst for liquefied petroleum gas synthesis during the synthesis of liquefied petroleum gas.

The yield of propane in liquefied petroleum gas produced by the present embodiment can be **e.g.** 15 Cmol% or higher, 20 Cmol% or higher, or further 24 Cmol% or higher, even at a low synthesis temperature (e.g. 330°C or lower).

The total yield of propane and butane (the combined total of the propane yield and the butane yield) produced by the present embodiment is **e.g.** 28 Cmol% or higher, preferably 30 Cmol% or higher.

Since especially the propane yield can be improved in the present embodiment, the volume ratio of the propane yield to the total yield of propane and butane can be **e.g.** 75% or higher, preferably 76% or higher, more preferably 80% or higher.

Next, the method for producing liquefied petroleum gas in the embodiment will be described.

The method for producing liquefied petroleum gas of the present embodiment includes a reduction treatment step, a supply step, and a synthesis step.

In the reduction treatment step, the above catalyst for liquefied petroleum gas synthesis is reduced. By the reduction treatment, copper oxide is reduced into copper. Preferably, the catalyst for liquefied petroleum gas synthesis is reduced with hydrogen.

In the supply step performed after the reduction treatment step, carbon monoxide (CO) and hydrogen (H₂) are supplied to the catalyst for liquefied petroleum gas synthesis reduced in the reduction treatment step. Carbon monoxide and hydrogen are both in gaseous form. Regarding the supply method, carbon monoxide and hydrogen may be supplied separately, or a mixed gas containing carbon monoxide and hydrogen, such as synthesis gas, may be supplied.

In the synthesis step performed after the supply step, carbon monoxide and hydrogen supplied in the supply step are reacted by the reduced catalyst for liquefied petroleum gas synthesis to synthesize liquefied petroleum gas. By using the above catalyst for liquefied petroleum gas synthesis to react carbon monoxide and hydrogen, the CO conversion rate, the propane and butane total yield, and the propane yield can be improved. Additionally, the catalyst for liquefied petroleum gas synthesis of the present embodiment is resistant to deterioration and excellent in long-term stability, and can exhibit good catalytic performance for a long period (e.g. 70 hours or longer at a synthesis temperature of 330°C or lower).

Regarding the gas hourly space velocity (GHSV) for supplying carbon monoxide and hydrogen in the supply step, the lower limit value is preferably 500/h or higher, more preferably 1000/h or higher, even more preferably 1500/h or higher, and the upper limit value is preferably 20000/h or lower, more preferably 10000/h or lower, even more preferably 5000/h or lower.

When the above gas hourly space velocity (GHSV) is 500/h or more, it is possible to efficiently produce liquefied petroleum gas from carbon monoxide and hydrogen. Additionally, when the above gas hourly space velocity (GHSV) is 20000/h or lower, it is possible to suppress the increase in the content ratio of unreacted substances such as carbon monoxide and hydrogen in the gas containing liquefied petroleum gas obtained after synthesis.

Regarding the temperature (synthesis temperature) of the catalyst in the synthesis step, the lower limit value is preferably 260°C or higher, more preferably 270°C or higher, even more preferably 280°C or higher, and the upper limit value is preferably 330°C or lower, more preferably 325°C or lower, even more preferably 320°C or lower.

In the synthesis step, by reacting carbon monoxide and hydrogen at a temperature of 260°C or more, it is possible to efficiently produce liquefied petroleum gas from carbon monoxide and hydrogen. Additionally, by reacting carbon monoxide and hydrogen at a temperature of 330°C or lower in the synthesis step, it is possible to suppress deterioration in the catalytic performance of the catalyst for liquefied petroleum gas synthesis due to temperature. Also, by reacting carbon monoxide and hydrogen at a temperature of 330°C or lower in the synthesis step, it is possible to suppress decrease in the yield due to excessive decomposition of the produced liquefied petroleum gas (e.g. decomposition from propane to ethane, or from ethane to methane).

Regarding the pressure in the synthesis step, the lower limit value is preferably 2.0 MPa or higher, more preferably 3.0 MPa or higher, even more preferably 3.5 MPa or higher, and the upper limit value is preferably 6.0 MPa or lower, more preferably 5.5 MPa or lower, even more preferably 5.0 MPa or lower, under which carbon monoxide and hydrogen are reacted.

By reacting carbon monoxide and hydrogen at a pressure of 2.0 MPa or higher in the synthesis step, it is possible to efficiently produce liquefied petroleum gas from carbon monoxide and hydrogen. Furthermore, by reacting carbon monoxide and hydrogen at a pressure of 6.0 MPa or lower in the synthesis step, it is possible to suppress deterioration in the catalytic performance of the catalyst for liquefied petroleum gas synthesis due to pressure.

The catalyst for liquefied petroleum gas synthesis can be produced e.g. by mixing the Cu-Zn based catalytic material and the MFI type zeolite catalytic material. The compositions, ratios, states, and the like of the Cu-Zn based catalytic material and the MFI type zeolite catalytic material are appropriately set depending on the desired liquefied petroleum gas.

The method for producing the Cu-Zn based catalytic material is not particularly limited, but can be exemplified by a coprecipitation method. In the coprecipitation method, a salt of metal components (copper, zinc, aluminium, zirconium, etc.) contained in the Cu-Zn based catalytic material, and a precipitant are used. Examples of the salt include nitrate, sulfate, acetate, and chloride. Examples of the precipitant include sodium carbonate and sodium bicarbonate.

Specifically, an aqueous solution of the salt of the metal components contained in the Cu-Zn based catalytic material and an aqueous solution of the precipitant are added dropwise into heated water and stirred to produce the Cu-Zn based catalytic material in a form of precipitate (slurry).

Concentration of the salt of each metal components in the aqueous solution of the salt of the metal components contained in the Cu-Zn based catalytic material should be set depending on the Cu-Zn based catalytic material to be produced.

After dropwise addition and stirring for about 10 to 180 minutes, the aqueous solution is further stirred for about 10 to 180 minutes to mature the precipitate (slurry). The progress of the reaction can be confirmed by checking pH. Preferably, pH of the reaction solution after the maturation (after completion of the reaction) is about 7.0 or higher to about 7.5. The dropwise addition and stirring should be performed while heating the reaction solution such that the temperature of the reaction solution is 50°C or higher and 80°C or lower.

The precipitate is then washed to remove Na and the like resulting from the precipitant, and dried to obtain the Cu-Zn based catalytic substance.

The above molar ratio (SiO₂/Al₂O₃) of the MFI type zeolite catalytic material can be controlled by, for example, adjusting the amount of aluminium source added during the synthesis of the zeolite catalytic material.

The amount of solid acid in the MFI type zeolite catalytic material can be controlled by, for example, adjusting the synthesis conditions (such as pH) during the synthesis of the zeolite catalytic material.

The method for supporting noble metal such as platinum on the MFI type zeolite catalytic material is not particularly limited, but such method includes an impregnation method, an immersion method, and an ion-exchange method.

When supporting platinum and palladium on the MFI type zeolite catalytic material, it is preferable to simultaneously support both platinum and palladium using an impregnation liquid or an immersion liquid containing platinum and palladium, rather than to sequentially support each metal through separate impregnation or immersion.

A compound containing platinum or palladium can be used as a starting material for platinum or palladium to be supported on the MFI type zeolite catalytic material. For the starting material of platinum, chloroplatinic acid hexahydrate, dinitrodiammine platinum, dichlorotetraammine platinum, platinum oxide, platinum chloride, and the like can be used. For the starting material of palladium, palladium chloride, palladium nitrate, dinitrodiammine palladium, palladium sulfate, palladium oxide, and the like can be used.

After impregnating the MFI type zeolite catalytic material with a solution of a compound containing platinum or palladium, such as a chloroplatinic acid solution or a palladium chloride solution, or after immersing the MFI type zeolite catalytic material in the solution of the compound containing platinum or palladium, the impregnated or immersed zeolite catalytic material can be calcined to efficiently highly disperse Pt or Pd in the MFI type zeolite catalytic material and to easily control the amount of Pt or Pd supported on the MFI type zeolite catalytic material.

The concentration of the compound containing platinum or palladium in the solution of the compound containing platinum or palladium should be set depending on the amount of platinum or palladium to be supported. For instance, the concentration of chloroplatinic acid hexahydrate in the solution is preferably 0.15 mass% or more and 3.50 mass% or less. The concentration of palladium chloride in the solution is preferably 0.1 mass% or more and 2.5 mass% or less. The supporting amount of Pt or Pd can be controlled depending on the concentration of the compound in the solution.

For sufficient penetration of platinum or palladium into the MFI type zeolite catalytic material, the impregnation time or the immersion time of the above solution is preferably 10 minutes or longer and 5 hours or shorter.

The calcination temperature of the MFI type zeolite catalytic material is preferably 300°C or higher and 600°C or lower, and the calcination time of the MFI type zeolite catalytic material is preferably 30 minutes or longer and 300 minutes or shorter.

The method for supporting phosphorus on the MFI type zeolite catalytic material is not particularly limited, but for example, an impregnation method or an immersion method can be used.

When supporting phosphorus on the MFI type zeolite catalytic material, orthophosphoric acid, phosphate ester, or the like can be used as a starting material for phosphorus. For example, an aqueous solution of orthophosphoric acid or phosphate ester can be used as an impregnation liquid or an immersion liquid.

Regarding the impregnation method or the immersion method when supporting phosphorus, after impregnating the MFI type zeolite catalytic material with a solution (hereinafter, also referred to as "phosphoric acid solution") of orthophosphoric acid or phosphate ester or after immersing the MFI type zeolite catalytic material in a phosphoric acid solution, the impregnated or immersed MFI type zeolite catalytic material is calcined, so that P can be efficiently incorporated into the MFI type zeolite catalytic material, and the amount of P contained in the MFI type zeolite catalytic material can be easily controlled.

The concentration of the phosphoric acid solution is preferably 2 mass% or more and 20 mass% or less.

For sufficient penetration of the phosphoric acid solution into the MFI type zeolite catalytic material, the impregnation time or the immersion time of the phosphoric acid solution is preferably 10 minutes or longer and 5 hours or shorter.

The calcination temperature of the MFI type zeolite catalytic material is preferably 300°C or higher and 600°C or lower. The calcination time of the MFI type zeolite catalytic material is preferably 30 minutes or longer and 300 minutes or shorter.

The content ratio of P can be controlled by adjusting the P concentration in the phosphoric acid solution, the impregnation time of the phosphoric acid solution or the immersion time of the phosphoric acid solution.

It is preferable to support the noble metal such as platinum or palladium on the MFI type zeolite catalytic material after incorporating P into the MFI type zeolite catalytic material. Specifically, for instance, it is preferable that, after impregnating or immersing the MFI type zeolite catalytic material with/in the phosphoric acid solution, the impregnated or immersed MFI type zeolite catalytic material is calcined, and the calcined MFI type zeolite catalytic material is impregnated or immersed with/in a solution containing the noble metal such as platinum or palladium, and then the impregnated or immersed MFI type zeolite catalytic material with/in the solution containing the noble metal such as platinum or palladium is calcined.

The liquefied petroleum gas produced using the above catalyst for liquefied petroleum gas synthesis contains a large amount of propane as its component, and can therefore be stably used as a fuel even in cold regions.

While embodiments have been described above, the present invention is not limited to the above embodiments but encompasses all modifications within the scope of the present disclosure and the claims, and can be modified in various ways within the scope of the present disclosure.

### EXAMPLES

Next, Examples and Comparative Examples will be described, but the present disclosure is not limited to these Examples.

### <Example 1>

### (Cu-Zn Based Catalytic Material)

First, 9.513 g of copper nitrate trihydrate, 4.973 g of zinc nitrate hexahydrate, 1.933 g of aluminium nitrate nonahydrate, and 0.531 g of zirconium nitrate dihydrate were dissolved in 58.4 g of distilled water to prepare Solution A. Additionally, 14.8 g of anhydrous sodium carbonate was dissolved in 200 g of distilled water to prepare Solution B.

A 500 ml separable flask with a stirrer was charged with 50 g of distilled water and heated in a water bath until the water temperature reached 65°C.

Subsequently, the entirety of Solution A and 90 ml of Solution B were added dropwise to the above separable flask after a rate of a liquid delivery pump was set such that the dropwise addition was completed in 70 minutes. Vigorous stirring was maintained during the dropwise addition. The precipitate slurry temperature was adjusted to 65°C with the water bath, as needed. After the dropwise addition of Solution A and Solution B, the pH was approximately 5.6**.** The remaining part of Solution B was then slowly added dropwise to the flask so as to bring the pH to 6.5**.** After the pH reached 6.5, the precipitate slurry was stirred vigorously at 65°C for 2 hours. The pH after 2 hours was approximately 7.2.

Subsequently, the precipitate slurry was transferred to a suction filtration apparatus and filtered to obtain a precipitate cake. The obtained precipitate cake was washed 20 times with 25 ml of distilled water to remove Na ion from the precipitate cake.

After washing, the precipitate cake was transferred to an evaporating dish and dried in a drying oven at 120°C for 12 hours. The cake was then heated in a calcination furnace at a rate of 10°C/min up to 350°C and calcined at 350°C for 2 hours, and the resulting calcined product was then thoroughly ground by an agate mortar to produce powder.

This powder was pelletized under a pressure of 5 MPa using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of Cu-Zn based catalytic material was obtained, which had a particle size of 300 to 500 µm, a granular shape, and a bulk density of 0.9 g/cm³.

As a result of measurement by ICP-OES, the Cu-Zn based catalytic material included copper oxide (CuO), zinc oxide (ZnO), zirconium oxide (ZrO₂), and aluminium oxide (Al₂O₃), and had a chemical composition with 62.6 mass% of copper oxide, 27.2 mass% of zinc oxide, 4.9 mass% of zirconium oxide, and 5.3 mass% of aluminium oxide.

### (MFI Type Zeolite Catalytic Material)

To an agate mortar, 36 g of MFI type zeolite (ZSM-5, molar number of SiO₂/molar number of Al₂O₃=40) was added, to which an aqueous solution with 2.3241 g of orthophosphoric acid dissolved in 21.6 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 30 g of the above calcined MFI type zeolite containing P was added, to which an aqueous solution with 0.4002 g of chloroplatinic acid hexahydrate dissolved in 22.7274 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pt. The MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 0.5 mass% of platinum ((M(Pt)/M2)×100 being 0.5 mass%), 1.9 mass% of phosphorus ((M(P)/M2)×100 being 1.9 mass%), with the remainder being ZSM-5.

### (Production of Liquefied Petroleum Gas)

For the catalyst for liquefied petroleum gas synthesis, a mixture of the molded body made of the Cu-Zn based catalytic material obtained as mentioned above and the molded body made of the MFI type zeolite catalytic material obtained as mentioned above was used. The mixing was performed so that the ratio (M1/(M1+M2)) of the mass (M1) of the Cu-Zn based catalytic material to the total mass of the mass (M1) of the Cu-Zn based catalytic material and the mass (M2) of the MFI type zeolite catalytic material was 0.5. Note that the M2 refers to the combined total of the mass of the supported noble metal (Pt), the mass of the MFI type zeolite catalytic material supporting the noble metal (Pt), and the mass of P. Then, liquefied petroleum gas was produced using the catalyst for liquefied petroleum gas synthesis under the following conditions.

First, the catalyst for liquefied petroleum gas synthesis was reduced with hydrogen. Subsequently, carbon monoxide and hydrogen were supplied to the catalyst for liquefied petroleum gas synthesis at a gas hourly space velocity (GHSV) of 2000/h. The temperature (synthesis temperature) was controlled to 320°C and the pressure was controlled to 5.0 MPa while supplying carbon monoxide and hydrogen to the catalyst for liquefied petroleum gas synthesis, to synthesize liquefied petroleum gas from carbon monoxide and hydrogen.

A fixed-bed high-pressure flow reactor was used for the reaction to synthesize liquefied petroleum gas from a mixed gas of carbon monoxide and hydrogen (CO:H₂=1:2 (molar ratio)). The reactor was made of stainless steel (inner diameter of 6.2 mm, total length of 60 cm). The center of the reactor was filled with the catalyst for liquefied petroleum gas synthesis wrapped with glass wool. The reactor was installed in an electric furnace, and a temperature of the electric furnace was measured by a thermocouple inserted into the center of the furnace and controlled by Proportional-Integral-Differential (PID) control. The temperature of the catalyst for liquefied petroleum gas synthesis was measured by a thermocouple inserted into the center of the catalyst layer in the reactor. The temperature of the catalyst for liquefied petroleum gas synthesis is the synthesis temperature. The catalyst for liquefied petroleum gas synthesis was reduced by supplying H₂ to the catalyst in the reactor with a flow rate of 40 ml/min at 380°C for 2 hours before the reaction.

### (Analysis of Gas Composition)

At a predetermined time point after the start of the reaction, the gas was analyzed using a gas chromatograph connected on-line. For the gas chromatograph, GC-2014 (Shimadzu Corporation) was used. The subjects and conditions for the analysis will be described below.

### <Analysis Condition>

| | |
|---|---|
| Column: RT-Q-BOND | |
| Temperature raising program: | (i) 45°C (held for 30 min) |
| | (ii) The temperature is raised to |
| 175°C at 2°C/min | (iii) 175°C (held for 40 min) |
| Analysis time 135 min | |

### [Measurement and Evaluation]

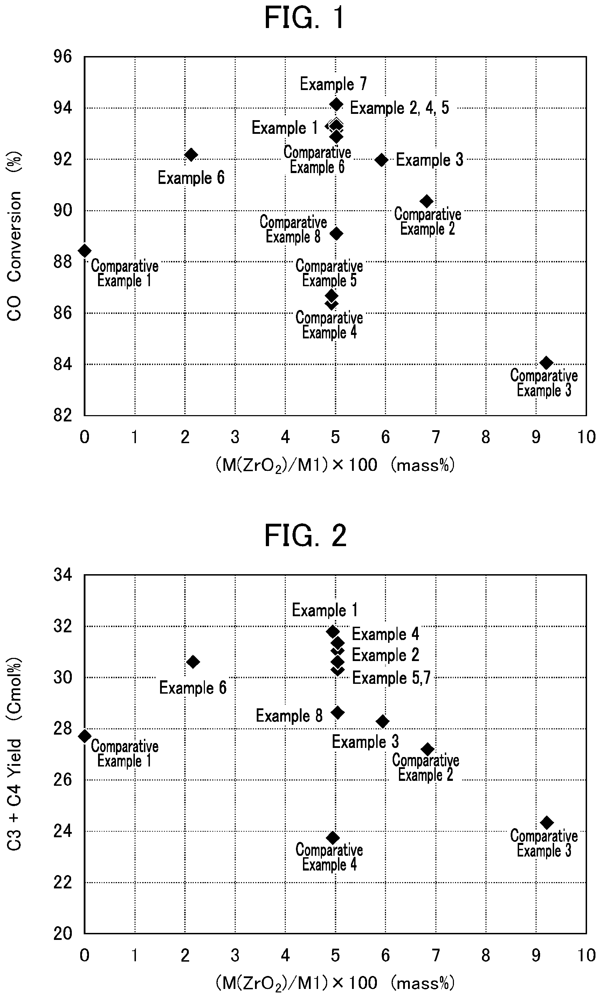

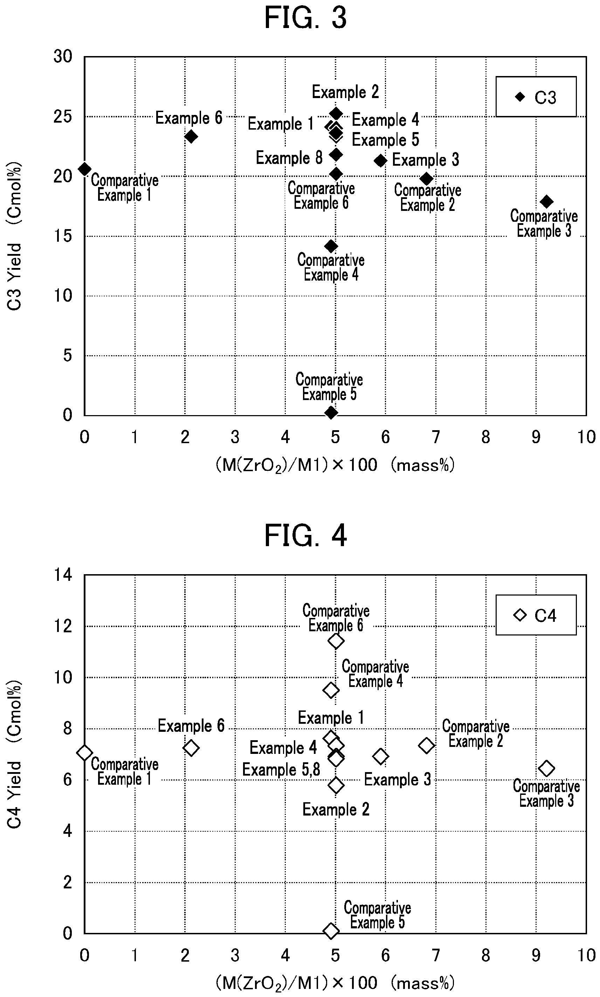

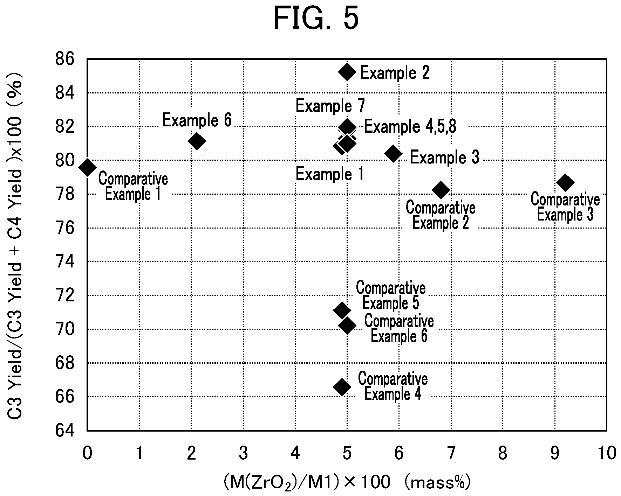

The catalyst for liquefied petroleum gas synthesis used in the Examples and Comparative Examples, and the liquefied petroleum gas obtained in above Examples and Comparative Examples were subjected to measurements and evaluations as below. The results are presented in Table 1. For Examples 1 to 8 and Comparative Examples 1 to 6, Fig. 1 presents the CO conversion rate, Fig. 2 presents the propane and butane total yield, Fig. 3 presents the propane yield, Fig. 4 presents the butane yield, and Fig. 5 presents the volume ratio of propane yield to the propane and butane total yield. The results for liquefied petroleum gas were obtained from the measurement 6 hours after the start of the reaction between carbon monoxide and hydrogen. Content ratio of zirconium oxide to the Cu-Zn based catalytic material (mass (M(ZrO2)) of zirconium oxide to mass (M1) of the Cu-Zn based catalytic material) (M(ZrO2)/M1)×100)

The content ratio of zirconium oxide to the Cu-Zn based catalytic material was measured by ICP-OES (Inductively Coupled Plasma Optical Emission Spectroscopy).Content ratio of aluminium oxide to the Cu-Zn based catalytic material (mass (M(Al2O3)) of aluminium oxide to mass (M1) of the Cu-Zn based catalytic material) (M(Al2O3)/M1)×100) (alumina)

The content ratio of aluminium oxide to the Cu-Zn based catalytic material was measured by ICP-OES (Inductively Coupled Plasma Optical Emission Spectroscopy). Mass (M(Pt)) of Pt to mass (M2) of the MFI type zeolite catalytic material ((M(Pt)/M2)x100)

The above ratio (M(Pt)/M2)×100 was measured by ICP-OES (Inductively Coupled Plasma Optical Emission Spectroscopy). Content ratio of P to the MFI type zeolite catalytic material (ratio of mass (M(P)) of P to mass (M2) of the MFI type zeolite catalytic material ((M(P)/M2)x100))

The content ratio of P to the MFI type zeolite catalytic material was measured by ICP-OES (Inductively Coupled Plasma Optical Emission Spectroscopy).

### [5] CO conversion rate (CO Conversion in Fig. 1)

CO conversion rate (%)=[(CO flow rate at the inlet (µmol/min)-CO flow rate at the outlet (µmol/min))/CO flow rate at the inlet (µmol/min)]×100

The CO conversion rate indicates a ratio of carbon monoxide (CO) converted into hydrocarbons and the like in the reaction raw gas.

### [6] Propane yield (C3 Yield in Fig. 2, Fig. 3, and Fig. 5)

Propane yield (Cmol%)=[(C3 production rate)/(CO flow rate at the inlet)×106/22400]×100

The unit of the C3 production rate is Cumol/min, and the unit of the CO flow rate at the inlet is ml (Normal)/min. C3 represents propane.

### [7] Butane yield (C4 Yield in Fig. 2, Fig. 4, and Fig. 5)

Butane yield (Cmol%)=[(C4 production rate)/(CO flow fate at the inlet)×106/22400]×100

The unit of the C4 production rate is Cumol/min, and the unit of the CO flow rate at the inlet is ml (Normal)/min. C4 represents butane.

### [8] Propane and butane total yield (C3+C4 Yield in Fig. 2 and Fig. 5)

Propane and butane total yield (Cmol%)=propane yield (Cmol%)+butane yield (Cmol%)

### [9] Volume ratio of propane yield to propane and butane total yield (C3/(C3+C4) ratio in Fig. 5).

Volume ratio of propane yield to propane and butane total yield=[molar number of propane/(molar number of propane+molar number of butane)]×100

### <Example 2>

The operations were carried out in the same manner as in Example 1, except that the amount of aluminium nitrate nonahydrate was changed to 1.896 g and the amount of zirconium nitrate dihydrate was changed to 0.542 g. The MFI type zeolite catalytic material in Example 1 was used.

In Example 2, the obtained Cu-Zn based catalytic material included copper oxide (CuO), zinc oxide (ZnO), aluminium oxide (Al₂O₃), and zirconium oxide (ZrO₂), and had a chemical composition with 62.6 mass% of copper oxide, 27.2 mass% of zinc oxide, 5.2 mass% of aluminium oxide, and 5.0 mass% of zirconium oxide.

### <Example 3>

The operations were carried out in the same manner as in Example 1, except that the amount of aluminium nitrate nonahydrate was changed to 1.565 g and the amount of zirconium nitrate dihydrate was changed to 0.640 g. The MFI type zeolite catalytic material in Example 1 was used.

In Example 3, the obtained Cu-Zn based catalytic material included copper oxide (CuO), zinc oxide (ZnO), aluminium oxide (Al₂O₃), and zirconium oxide (ZrO₂), and had a chemical composition with 62.6 mass% of copper oxide, 27.2 mass% of zinc oxide, 4.3 mass% of aluminium oxide, and 5.9 mass% of zirconium oxide.

### <Example 4>

The operations were carried out in the same manner as in Example 2, except that the preparation method for the MFI type zeolite catalytic material was changed to the following method.

### (MFI Type Zeolite Catalytic Material)

To an agate mortar, 12 g of MFI type zeolite (ZSM-5, molar number of SiO₂/molar number of Al₂O₃=40) was added, to which an aqueous solution with 0.7747 g of orthophosphoric acid dissolved in 7.2 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 10 g of the above calcined MFI type zeolite containing P was added, to which an aqueous solution with 0.2682 g of chloroplatinic acid hexahydrate dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pt. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 1.0 mass% of platinum ((M(Pt)/M2)×100 being 1.0 mass%), 1.95 mass% of phosphorus ((M(P)/M2)×100 being 1.93 mass%), with the remainder being ZSM-5.

### <Example 5>

The operations were carried out in the same manner as in Example 2, except that the preparation method for the MFI type zeolite catalytic material was changed to the following method.

### (MFI Type Zeolite Catalytic Material)

To an agate mortar, 12 g of MFI type zeolite (ZSM-5, molar number of SiO₂/molar number of Al₂O₃=40) was added, to which an aqueous solution with 0.7747 g of orthophosphoric acid dissolved in 7.2 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 10 g of the above calcined MFI type zeolite containing P was added, to which an aqueous solution with 0.5418 g of chloroplatinic acid hexahydrate dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pt. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 2.0 mass% of platinum ((M(Pt)/M2)×100 being 2.0 mass%), 1.91 mass% of phosphorus ((M(P)/M2)×100 being 1.91 mass%), with the remainder being ZSM-5.

### <Example 6>

The operations were carried out in the same manner as in Example 1, except that the amount of aluminium nitrate nonahydrate was changed to 2.963 g and the amount of zirconium nitrate dihydrate was changed to 0.228 g. The MFI type zeolite catalytic material in Example 1 was used.

In Example 6, the obtained Cu-Zn based catalytic material included copper oxide (CuO), zinc oxide (ZnO), aluminium oxide (Al₂O₃), and zirconium oxide (ZrO₂), and had a chemical composition with 62.6 mass% of copper oxide, 27.2 mass% of zinc oxide, 8.1 mass% of aluminium oxide, and 2.1 mass% of zirconium oxide.

### <Example 7>

The operations were carried out in the same manner as in Example 2, except that the preparation method for the MFI type zeolite catalytic material was changed to the following method.

### (MFI Type Zeolite Catalytic Material)

To an agate mortar, 12 g of MFI type zeolite (ZSM-5, molar number of SiO₂/molar number of Al₂O₃=40) was added, to which an aqueous solution with 0.3834 g of orthophosphoric acid dissolved in 7.2 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 10 g of the above calcined MFI type zeolite containing P was added, to which an aqueous solution with 0.1334 g of chloroplatinic acid hexahydrate dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pt. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 0.5 mass% of platinum ((M(Pt)/M2)×100 being 0.5 mass%), 1.0 mass% of phosphorus ((M(P)/M2)×100 being 1.0 mass%), with the remainder being ZSM-5.

### <Example 8>

The operations were carried out in the same manner as in Example 2, except that the preparation method for the MFI type zeolite catalytic material was changed to the following method.

### (MFI Type Zeolite Catalytic Material)

To an agate mortar, 12 g of MFI type zeolite (ZSM-5, molar number of SiO₂/molar number of Al₂O₃=40) was added, to which an aqueous solution with 1.1740 g of orthophosphoric acid dissolved in 7.2 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 10 g of the above calcined MFI type zeolite containing P was added, to which an aqueous solution with 0.1334 g of chloroplatinic acid hexahydrate dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pt. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 0.5 mass% of platinum ((M(Pt)/M2)×100 being 0.5 mass%), 2.9 mass% of phosphorus ((M(P)/M2)×100 being 2.9 mass%), with the remainder being ZSM-5.

### <Comparative Example 1>

The operations were carried out in the same manner as in Example 1, except that the amount of aluminium nitrate nonahydrate was changed to 3.736 g, and zirconium nitrate dihydrate was not used.

In Comparative Example 1, the obtained Cu-Zn based catalytic material included copper oxide (CuO), zinc oxide (ZnO), and aluminium oxide (Al₂O₃), and had a chemical composition with 62.6 mass% of copper oxide, 27.2 mass% of zinc oxide, and 10.2 mass% of aluminium oxide.

### <Comparative Example 2>

The operations were carried out in the same manner as in Example 1, except that the amount of aluminium nitrate nonahydrate was changed to 1.234 g and the amount of zirconium nitrate dihydrate was changed to 0.738 g.

In Comparative Example 2, the obtained Cu-Zn based catalytic material included copper oxide (CuO), zinc oxide (ZnO), aluminium oxide (Al₂O₃), and zirconium oxide (ZrO₂), and had a chemical composition with 62.6 mass% of copper oxide, 27.2 mass% of zinc oxide, 3.4 mass% of aluminium oxide, and 6.8 mass% of zirconium oxide.

### <Comparative Example 3>

The operations were carried out in the same manner as in Example 1, except that aluminium nitrate nonahydrate was not used, and the amount of zirconium nitrate dihydrate was changed to 0.998 g.

In Comparative Example 3, the obtained Cu-Zn based catalytic material included copper oxide (CuO), zinc oxide (ZnO), aluminium oxide (Al₂O₃), and zirconium oxide (ZrO₂), and had a chemical composition with 62.6 mass% of copper oxide, 27.2 mass% of zinc oxide, 1.0 mass% of aluminium oxide, and 9.2 mass% of zirconium oxide.

### <Comparative Example 4>

The operations were carried out in the same manner as in Example 1, except that the preparation method for the MFI type zeolite catalytic material was changed to the following method.

### (MFI Type Zeolite Catalytic Material)

To an agate mortar, 12 g of MFI type zeolite (ZSM-5, molar number of SiO₂/molar number of Al₂O₃=40) was added, to which an aqueous solution with 0.7747 g of orthophosphoric acid dissolved in 7.2 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 10 g of the above calcined MFI type zeolite containing P was added, to which an aqueous solution with 0.0837 g of palladium chloride dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pd. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pd was obtained, which had a particle size of 300 to 500 µm, a granular shape, and a bulk density of 0.8 g/cm³.

As a result of measurement by ICP-OES, the MFI type zeolite catalytic material had a chemical composition with 0 mass% of platinum ((M(Pt)/M2)×100 being 0 mass%), 0.5 mass% of palladium, and 1.9 mass% of phosphorus ((M(P)/M2)×100 being 1.9 mass%), with the remainder being ZSM-5.

### <Comparative Example 5>

The operations were carried out in the same manner as in Example 1, except that the preparation method for the MFI type zeolite catalytic material was changed to the following method.

### (MFI Type Zeolite Catalytic Material)

To an agate mortar, 12 g of MFI type zeolite (ZSM-5, molar number of SiO₂/molar number of Al₂O₃=40) was added, to which an aqueous solution with 1.9979 g of orthophosphoric acid dissolved in 7.2 g of pure water was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes, and calcined while maintaining this temperature for 120 minutes, under air atmosphere.

To an agate mortar, 10 g of the above calcined MFI type zeolite containing P was added, to which an aqueous solution with 0.1334 g of chloroplatinic acid hexahydrate dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite containing P and supporting Pt. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material containing P and supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 0.5 mass% of platinum ((M(Pt)/M2)×100 being 0.5 mass%), 4.7 mass% of phosphorus ((M(P)/M2)×100 being 4.7 mass%), with the remainder being ZSM-5.

### <Comparative Example 6>

The operations were carried out in the same manner as in Example 2, except that the preparation method for the MFI type zeolite catalytic material was changed to the following method.

### (MFI Type Zeolite Catalytic Material)

Under air atmosphere, 12 g of MFI type zeolite (ZSM-5, molar number of SiO₂/molar number of Al₂O₃=40) was heated from normal temperature to 500°C for 50 minutes and calcined while maintaining this temperature for 120 minutes.

To an agate mortar, 10 g of the above calcined MFI type zeolite was added, to which an aqueous solution with 0.1334 g of chloroplatinic acid hexahydrate dissolved in 7.5758 g of 10% hydrochloric acid was added dropwise using a pipette while uniformly mixing them using a pestle for 1 hour to impregnate the MFI type zeolite with the solution. Afterwards, the MFI type zeolite was dried at 100°C for 10 hours, then heated from normal temperature to 500°C for 50 minutes and calcined while maintaining this temperature for 120 minutes under air atmosphere, to obtain an MFI type zeolite supporting Pt. Subsequently, the MFI type zeolite was pelletized using a tablet press to produce pellets with a diameter of 20 mm and a thickness of about 1 mm, then the pellets were crushed by a mortar, and the crushed sample was sieved using overlaid 300 µm and 500 µm mesh sieves. As a result, a molded body made of the MFI type zeolite catalytic material supporting Pt was obtained, which had a particle size of 300 to 500 µm, a granular shape, and a bulk density of 0.8 g/cm³.

As a result of measurement by ICP-OES, the MFI type zeolite catalytic material had a chemical composition with 0.5 mass% of platinum ((M(Pt)/M2)×100 being 0.5 mass%), 0 mass% of phosphorus ((M(P)/M2)×100 being 0 mass%), with the remainder being ZSM-5.

**[Table 1]**

| | Example 1 | Example 2 | Example 3 | Example 4 | Example 5 | Example 6 | Example 7 | Example 8 | Comparative Example 1 | Comparative Example 2 | Comparative Example 3 | Comparative Example 4 | Comparative Example 5 | Comparative Example 6 |
|---|---|---|---|---|---|---|---|---|---|---|---|---|---|---|
| (M(ZrO₂)/M1)×100 (mass%) | 4.9 | 5.0 | 5.9 | 5.0 | 5.0 | 2.1 | 5.0 | 5.0 | 0 | 6.8 | 9.2 | 4.9 | 4.9 | 5.0 |
| Alumina (mass%) | 5.3 | 5.2 | 4.3 | 5.2 | 5.2 | 8.1 | 5.2 | 5.2 | 10.2 | 3.4 | 1.0 | 5.3 | 5.3 | 5.2 |
| **Pt** (mass%) | 0.5 | 0.5 | 0.5 | 1.0 | 2.0 | 0.5 | 0.5 | 0.5 | 0.5 | 0.5 | 0.5 | 0 | 0.5 | 0.5 |
| P (mass%) | 1.9 | 1.9 | 1.9 | 1.9 | 1.9 | 1.9 | 1.0 | 2.9 | 1.9 | 1.9 | 1.9 | 1.9 | 4.7 | 0.0 |
| CO conversion rate (%) | 93.3 | 93.4 | 92.0 | 93.2 | 93.3 | 92.2 | 94.2 | 89.1 | 88.4 | 90.4 | 84.1 | 86.4 | 86.7 | 92.9 |
| Propane and butane total yield (Cmol%) | 31.8 | 31.1 | 28.3 | 31.4 | 30.3 | 30.6 | 30.6 | 28.6 | 27.7 | 27.2 | 24.3 | 23.8 | 0.37 | 31.7 |
| Propane yield (Cmol%) | 24.2 | 25.3 | 21.4 | 24.0 | 23.4 | 23.4 | 23.7 | 21.8 | 20.7 | 19.9 | 17.9 | 14.2 | 0.24 | 20.3 |
| Butane yield (Cmol%) | 7.6 | 5.8 | 6.9 | 7.4 | 6.9 | 7.2 | 6.9 | 6.8 | 7.1 | 7.4 | 6.4 | 9.5 | 0.13 | 11.4 |
| Volume ratio of propane yield to propane and butane total yield (%) | 80.9 | 85.3 | 80.4 | 81.3 | 81.8 | 81.2 | 82.0 | 81.0 | 79.6 | 78.3 | 78.7 | 66.5 | 71.1 | 70.3 |

As presented in Table 1 and Fig. 1 to Fig. 5, Examples 1 to 8, which included the Cu-Zn based catalytic material and the MFI type zeolite catalytic material supporting Pt, the Cu-Zn based catalytic material contained copper oxide, zinc oxide, aluminium oxide, and zirconium oxide, the mass (M(ZrO₂)) of zirconium oxide in the Cu-Zn based catalytic material was more than 0 mass% and 6.5 mass% or less based on the mass (M1) of the Cu-Zn based catalytic material, and the MFI type zeolite catalytic material contained more than 0 mass% and less than 4.5 mass% of P, had higher the CO conversion rate, the propane and butane total yield, and the propane yield than Comparative Example 1 which did not contain zirconium oxide, Comparative Examples 2 and 3 in which the mass (M(ZrO₂)) of zirconium oxide was out of a range of more than 0 mass% and 6.5 mass% or less based on the mass (M1) of the Cu-Zn based catalytic material, Comparative Example 4 which did not support Pt, Comparative Example 5 in which the content ratio of P was out of a range of more than 0 mass% and less than 4.5 mass%, and Comparative Example 6 which did not contain P.

## Claims

1. A catalyst for liquefied petroleum gas synthesis, comprising a Cu-Zn based catalytic material and an MFI type zeolite catalytic material supporting Pt,
the Cu-Zn based catalytic material containing copper oxide, zinc oxide, aluminium oxide, and zirconium oxide,
a mass (M(ZrO₂)) of zirconium oxide in the Cu-Zn based catalytic material being more than 0 mass% and 6.5 mass% or less based on a mass (M1) of the Cu-Zn based catalytic material, and
the MFI type zeolite catalytic material containing more than 0 mass% and less than 4.5 mass% of P.

2. The catalyst for liquefied petroleum gas synthesis according to claim 1, wherein a mass (M(Al₂O₃)) of the aluminium oxide in the Cu-Zn based catalytic material is 3.5 mass% or more and 8.0 mass% or less based on the mass (M1) of the Cu-Zn based catalytic material.

3. The catalyst for liquefied petroleum gas synthesis according to claim 1, wherein a ratio (M1/(M1+M2)) of the mass (M1) of the Cu-Zn based catalytic material to a total mass (M1+M2) of the mass (M1) of the Cu-Zn based catalytic material and the mass (M2) of the MFI type zeolite catalytic material is 0.30 or higher and 0.95 or lower.

4. The catalyst for liquefied petroleum gas synthesis according to claim 1, wherein a mass (M(Pt)) of Pt in the MFI type zeolite catalytic material is 0.1 mass% or more and 10.0 mass% or less based on the mass (M2) of the MFI type zeolite catalytic material.

5. The catalyst for liquefied petroleum gas synthesis according to claim 1, wherein
the Cu-Zn based catalytic material and the MFI type zeolite catalytic material exist independently from each other, and
both of the Cu-Zn based catalytic material and the MFI type zeolite catalytic material are in a form of granulated powder or molded body.

6. A method for producing liquefied petroleum gas comprising:
a reduction treatment step of reducing the catalyst for liquefied petroleum gas synthesis according to any one of claims 1 to 5;
a supply step of supplying carbon monoxide and hydrogen to the catalyst for liquefied petroleum gas synthesis reduced in the reduction treatment step; and
a synthesis step of synthesizing liquefied petroleum gas by reacting carbon monoxide and hydrogen supplied in the supply step using the catalyst for liquefied petroleum gas synthesis subjected to the reduction treatment.

7. The method for producing liquefied petroleum gas according to claim 6, wherein a gas hourly space velocity (GHSV) for supplying carbon monoxide and hydrogen is 500/h or higher and 20000/h or lower in the supply step.

8. The method for producing liquefied petroleum gas according to claim 6, wherein carbon monoxide and hydrogen are reacted at a temperature of 260°C or higher and 330°C or lower in the synthesis step.

9. The method for producing liquefied petroleum gas according to claim 6, wherein carbon monoxide and hydrogen are reacted under a pressure of 2.0 MPa or higher and 6.0 MPa or lower in the synthesis step.
